# EUROPEAN PATENT APPLICATION

(11) **EP 1 266 840 A2**
(43) Date of publication of application: **18.12.2002**
(21) Application number: 02012859.1
(22) Date of filing: 11.06.2002
(51) Int. Cl.: B65D 47/18

(54) **A dispensing container**

(30) Priority: 15.06.2001 JP 2001181404; 28.02.2002 JP 2002052839
(71) Applicant: Taisai Kako Co., Ltd., Osaka 531-0073 (JP)
(72) Inventor: Hamamoto, Keiji, Ibaraki-shi, Osaka 567-0054 (JP)
(74) Representative: Paul, Dieter-Alfred, Dipl.-Ing.

(57) **Abstract**

A dispensing container comprises a delaminating bottle (2) as a member for shutting off a liquid content from ambient air so that, without aid of any preservatives, a liquid residue is protected from contamination at a region adjacent to an outlet hole (3a). The container has a flexible nozzle (31) and a rod-shaped closer (32), both disposed in the mouth of bottle (2). The nozzle (31) is made of a pliable and elastic material, with the closer (32) being shaped to close a distal end region of the outlet hole (3a). By squeezing the barrel of bottle to increase its internal pressure, the flexible nozzle (31) expands to make a deformation, which in turn causes the outlet hole (3a) to take an outward open position.

## Description

### FIELD OF THE INVENTION

The present invention relates to a dispensing container adapted for packaging eye drops in aseptic condition, packaging some cosmetic liquids free of any preservatives, bactericides, antiseptics or the like pasteurizing agents, or packaging any other similar liquid products.

### BACKGROUND OF THE INVENTION

There are known some types of plug assemblies to be attached to the mouths of containers filled with any liquids. The known plug assemblies comprise each a dispensing valve that will remain closed so long as the internal pressure in the container is balanced with atmospheric pressure. Examples of such plug assemblies are disclosed in some Japanese Patent Laying-Open Gazettes No. 8-282704, No. 8-282703, or in the US Patents No. 5,213,236, No. 5,339,995 and No. 5,439,143.

Such conventional plug assemblies may preferably be used in certain dispensing containers for eye drops, liquid detergents or foods. Contents of these containers will surely be protected from leakage and allowed to smoothly exude when necessary.

Internal pressure will decrease as the content is discharged through the plug assembly, and will become lower than atmospheric pressure after every dosage. A dispensing valve incorporated in said plug assembly will thus open inwards to suck an amount of ambient air to cancel such a negative pressure. Since a variety of pathogenic microbes such as true fungi and viruses are present in the ambient air, certain preservatives have been added to those liquid contents so as to assure safe usage thereof.

However, as widely known in the art, preservatives have produced harmful side effects. It has been reported that eye drops with preservatives would cause inflammation or the like damage of the eyes, if used repeatedly. Contact lens-wearers sometimes suffer from allergic reactions even against some kinds of preservatives of a considerably low concentration. Hair detergents also contain such a preservative as causing inflammation of head skin and/or loss of hair. Further, many of recent consumers have in general a dislike to addition of preservatives to any products whatever.

Therefore, certain proposals were made to selectively remove only preservatives and other additives of no medical effects, from a liquid con-tent flowing out of a bottle. According to one of those proposals, a filter for removing preservatives is disposed in a flow channel formed through an eyedropper, as disclosed in the Japanese Laying-Open Gazette No. 4-297264 or No. 6-14972. Such a known eyedropper will however fail to diminish concentration of preservatives in every dosage of eyedrops, if the preservatives are contained so rich in a fluid content as to enhance sterilization effect. Those known eyedroppers are designed to permit in-flow of ambient air contaminated with microbes, presuming the preservatives at a considerable concentration thereof to be sufficiently disinfectant. In such a case, concentration of the preservatives cannot necessarily be reduced well to avoid any residual amount of them from remaining in the drops of eyewash having flown through the filter.

Utility Model Laying-Open Gazette No. 63-184037 discloses a hydrophilic filter membrane disposed in a discharging channel. This membrane permits through-flow of eye drops liquid from a container body but pre-vents inward permeation of bacteria and air. A distal end portion of the container body is of a depressed tubular or otherwise foldable shape so that effective volume of said body will reduce in response to decreased quantity of the eyewash remaining therein. In the eyedropper of this type, ambient air is shut out in order to prevent bacteria from entry to the container during use thereof. Concentration of preservatives can thus be rendered as low as possible in the fluid medicine. However, the container body is of such a single-layer structure as enabling its plastic deformation. It is difficult for any known plastic materials to form this container body, and therefore a certain proper aluminum tube must be used to make it, failing to afford transparency of the container. Such a depressed container cannot stand itself and will not be convenient for succeeding uses thereof.

Japanese Patent Laying-Open Gazettes No. 9-175566 and No. 10-165222 disclose a dispensing container that comprises a delaminating bottle having a mouth to which a plug assembly is attached. This bottle is composed of an outer cylindrical layer and an inner layer laminated thereon to be capable of reversibly peeling from the outer layer. The interior of inner layer will function as a reservoir for a liquid content, and the outer layer has an vent formed therein to suck ambient air. A channel or passage is formed through the plug assembly for exuding a liquid content stored in the reservoir. A check valve disposed in the flow passage will inhibit back-flow of the content accompanied by ambient air into the reservoir. But, the ambient air is sucked sideways through the vent to flow in between the layers, with the inner layer consequently shrinking as the liquid content is gradually forced out and dispensed. A major portion of a liquid content staying in the reservoir will be kept unspoiled with bacteria or the like, without aid of any amount of preservatives. However, a small quantity of liquid content will inevitably remain between the check valve and a dispensing outermost opening. Such a residual amount of liquid content will be contaminated with true fungi and/or viruses, in the course of time and before the next use.

### SUMMARY OF THE INVENTION

An object of the present invention made in view of the drawbacks inherent in the prior art structures is therefore to provide an improved dispensing container that comprises a delaminating bottle having a mouth to which a plug assembly is attached. The plug assembly in the present invention has to comprise a valvular nozzle mechanism that has an outlet opening formed therein to afford a sealing interruption between ambient air and a liquid content stored in the delaminating bottle, during non-operation thereof. Residual quantity of a liquid content remaining downstream of the outlet opening after every use of this container should be diminished herein to avoid the dispensing of a fresh dose contaminated with polluted residue.

A dispensing container of the present invention comprises a bottle, an interior bag of the bottle and a plug assembly. The bottle may comprise a barrel and a mouth. The bag may be containable a liquid content. The bag may be placed in the barrel of the bottle. The plug assembly may be disposed in the mouth of said bottle. The outlet opening may be connected to the mouth of said bottle. Preferably, an open end of the outlet opening may be closely connected all around to an open end of the mouth of the bottle. The interior bag is capable of deflating as its liquid content decreases in volume. The outlet opening may be adhered all around to an inner periphery of the mouth to thereby inhibit deformation of the adhered portion of the interior bag.

The bottle may have at least one vent for intake of ambient air in between the bottle and the interior bag. A plurality of such vents may either be formed in place of the mouth. The vent may be formed in a sidewall of the barrel or a bottom of the bottle. A check valve may be disposed in each of vents. Alternatively, check valves may not be built in said vents to render same normally open, thereby causing users to close those vents with their fingers when using this container.

The plug assembly may comprise a flexible nozzle having an outlet hole for dispensing the liquid content out of the bottle, and a closer capable of closing the outlet hole. The closer may be fixed in the mouth of the bottle, and the nozzle may be capable of elastic deformation away from a normal position such that the outlet hole is displaced outwards relative to the closer in response to a pressure raised in the bag and disengage from the closer.

In operation, the users may let the flexible nozzle face downwards and depress the exterior bottle to directly compress the interior bag filled with a liquid content, or otherwise compress the air intervening between said bottle and the bag that has then shrunk itself more or less. The internal pressure thus raised in the interior bag will cause the flexible nozzle to elastically deform itself so that its outlet hole moves outwards relative to the closer until disengagement therefrom. As a result, the outlet hole of the nozzle in liquid communication with the interior bag will open to dispense the liquid content out of this bag. Once the user ceases to depress the exterior bottle, it will instantly start to restore its normal shape due to elasticity. Thus, the vents will allow an amount of ambient air to enter a cavity between the bag and bottle, with the flexible nozzle simultaneously moving towards its normal position so that its outlet hole consequently creeps back inwards to be tightly closed again with the closer. It will now be apparent that the interior bag shrinks gradually and more or less whenever the user dispenses its liquid content to any desired target objects. Also importantly, the flexible nozzle cooperates at the same time with the closer to function like a check valve, lest any amount of ambient air should flow into the interior bag. Any preservatives need no longer be added to the liquid content stored in this interior bag. The vents permit introduction of a neat amount of ambient air into the annular cavity between the interior bag and the exterior bottle, normally keeping air pressure at atmospheric level around said bag. Thanks to this feature, the exterior bottle temporarily depressed for dispensation of liquid content will readily and smoothly regain its natural form, and this form will be maintained until complete exhaustion of said liquid content.

In the dispensing container of the invention, the closer may be a generally rod-shaped valve body, viz., 'needle' (as in usual needle valves) of a 'given length' inserted in the outlet hole so as to normally close it. The inner peripheral surface of the flexible nozzle is designed to temporarily produce a negative pressure in interior bag. Such a negative pressure appearing in this bag will act on the nozzle to be retracted inwardly towards the container's bottom. An inner region of interface present between the nozzle inner surface and the needle periphery stands always in fluid communication with the interior of the bag. Air-tightness and liquid-tightness are never broken between the nozzle and the closer, even if the nozzle's outlet hole closed with the needle (as the closer) might occasionally be displaced along it within a certain range. Thus, neither any inadvertent inflow of ambient air into, nor any inadvertent effluence of liquid content out of, the interior bag is likely to take place. The flexible nozzle moving, after one dispensing operation, towards its normal position to retract its outlet hole may possibly encounter a frictional resistance or the like caused by the needle or any other happening. Even in such an event, the flexible nozzle having the smooth inner periphery will be retained at an inner position where its outlet hole remains closed with the needle (i.e., rod-shaped valve body), by virtue of a negative pressure produced in the interior bag. This motion will be effected in unison with the inflow of ambient air through the vents. Internal pressure of the bag may unintentionally rise within a considerable range due to raised temperatures or due to vibration or shock when carrying the container. Such an inadvertently raised internal pressure will however be cancelled by the flexible nozzle's outward displacement relative to the needle but not beyond the 'given length' thereof (noted hereinbefore). Thus, the outlet hole remains closed not to cause any undesirable discharge of liquid content during storage or handling of the container.

The flexible nozzle may be made of any pliable and elastic material such as rubbers. It is not necessary for the nozzle to be flexible in its entirety, but it may have an elastically deforming thinned portion. Consequently, a thicker base of the flexible nozzle may be formed as a cylindrical part fitting in the exterior bottle's mouth. A sleeve may be disposed in such a base within the bottle mouth so that this portion is strongly and firmly gripped by and between the sleeve and the mouth inner periphery. Air-tightness and liquid-tightness all around the base of flexible nozzle are thus enhanced. Typically, the closer formed as the rod-shaped valve body (viz., needle) may be formed integral with such a sleeve. In this case, simple installment of the sleeve suffices well to fix both the cooperating flexible nozzle and closer in place, thus simplifying structure and reducing the number of component parts is carried out.

The plug assembly may comprise a sleeve made integral with the closer and fitted in the mouth of the bottle, the nozzle formed of a pliable and elastic material such as a rubber has a cylindrical base, and the base surrounds the closer and is in an airtight and liquid-tight contact with the sleeve. In this case, further simplifying structure and simplification of assembly process is carried out.

Additionally, a cap may be employed in combination with the already discussed parts of the dispensing container. The cap detachably mounted on the mouth of exterior bottle will cover an exterior surface of the flexible nozzle. The cap may have an inner periphery adjacent to top of the cap, wherein the periphery is substantially in conformity with upper end regions which the nozzle and the closer define in state what the cap is mounted on the mouth and the cap in the mounted state does closely contact end faces of the upper end regions. After every use of this dispensing container, the cap will be attached thereto so as to press inwards the outwards-facing end of an opening defining the outlet hole in said flexible nozzle. Any residual amount of liquid content remaining on such an outlet hole end will thus be forced off this hole, so as not give rise to the problem of contamination of said residual liquid. Preferably and typically, the flexible nozzle has a (bill-shaped) cylindrical nozzle portion protruding outwards and longitudinally of the container, with a bore of this cylindrical portion serving as the outlet hole. Also in this case, the cap has an upper portion whose inner periphery coincides with and closely contacts all around the bill-shaped nozzle portion. Any residual amount of liquid content adhering to outlet hole will be pushed down away from this hole's end towards a lower end of the bill-shaped portion. Preferably, the end of nozzle's outlet hole may be formed in flush with the upper end of closer, and the cap has at its top an inner surface in a plane contact with both the outlet hole's end and the closer's upper end. In this case, a residual amount of liquid content adhering to outlet hole can more surely be forced off the opening end of said outlet hole.

Also preferably, the barrel of exterior bottle is capable of elastic deformation to reversible reduce its volume. Compression of the interior bag will be caused by such an exterior bottle to exude the liquid content through the flexible nozzle outlet hole at its elastically deformed position. When the barrel subsequently regains its normal shape, the flexible nozzle returns to its normal position and the ambient air sucked through the vents flows in between the bottle and bag.

### BRIEF DESCRIPTION OF THE DRAWINGS

Fig. 1(a) is a plan view of a dispensing container provided in a first embodiment of the present invention;
Fig. 1(b) is a cross section taken along the line A - A in Fig. 1(a);
Fig. 2 is a cross section taken along the line B - B in Fig. 1(a);
Fig. 3(a) is a plan view of a dispensing container provided in a second embodiment;
Fig. 3(b) is a cross section taken along the line C - C in Fig. 3(a);
Fig. 4 is a cross section taken along the line D - D in Fig. 3(a);
Fig. 5(a) is a plan view of a dispensing container provided in a third embodiment;
Fig. 5(b) is a cross section taken along the line E - E in Fig. 5(a);
Fig. 6 is a cross section taken along the line F - F in Fig. 5(a); and
Fig. 7 is an enlarged, fragmentary and vertical cross section of a dispensing container provided in a fourth embodiment.

### THE PREFERRED EMBODIMENTS

The dispensing container of the present invention may be manufactured by previously forming an exterior bottle and an interior bag, and then fixing the latter in the former. However, it is more preferable that the present container is composed mainly of a delaminating bottle whose inner layer as the interior bag is laminated on an outer layer serving as the exterior bottle.

Also preferably installed in each of vents is a check valve that will permit inflow of ambient air into a clearance between the exterior bottle and interior bag. The check valve inhibits outflow of the ambient air having entered the clearance. Thanks to such a check valve, the ambient air in this clearance or gap will be compressed by squeezing the exterior bottle, to thereby raise air pressure therein. As a result, an amount of liquid content corresponding to the degree of compression of interior bag will flow out through a discharging channel or passage. If no check valves are used, then a user of this container need to close the vents when he or she squeezes the exterior bottle.

The laminated delaminating bottle may be molded to have a mouth and a barrel continuing thereto. Any proper method such as the injection-blow molding, the direct blow molding or the injection molding method can be employed for this purpose. An inner layer preform and an outer layer preform may be produced previously and discretely so that the former is inserted in the latter to prepare a laminated parison. In this case, the parison will be subjected to the blow molding process to give a container of any desired shape. The resulting container will be of a laminated structure such that both the inner and outer layers extend from top to bottom of said container. A laminated mouth will thus continue from a similarly laminated barrel. In an alternative mode, a previously molded film-like interior bag is placed in a raw exterior bottle having the mouth and barrel, before subjected to a similar finishing process.

The mouth is rendered higher in rigidity, by thickening both the exterior bottle and interior bag at this region. On the other hand in the barrel portion, the exterior bottle (viz., outer layer) should be made capable of squeezing and elastic recovery. The interior bag (viz., inner layer) has to readily shrink, like usual films, following decrease in volume of the liquid content. Each of exterior bottle and interior bag may either be of a single-layer or of a multi-layer structure.

The interior bag can be formed of any plastics such as a polyethylene (PE), a polypropylene (PP) or any other polyolefin. The exterior bottle can be molded using the other type of plastics such as saturated polyesters including a polyethylene terephthalate (PET) and a polyethylene naphthalate (PEN). In case of using the container as an eyedropper, it is compulsory that a proper raw material is selected to forming each of these exterior bottle and interior bag to render the container transparent and non-permeable to water. Because the interior bag directly contacts pharmaceutical contents, it must be made from a certain plastics (for example a PE) highly resistant to chemicals. In contrast, higher transparency and lower water permeation are properties desirable for the plastics as the material (e.g., a PET or a soft glass) of exterior bottle.

The vents may be formed either in the mouth, in the barrel or in a bottom portion of the exterior bottle. The check valve preferably disposed in each vent can be of any proper structure. For example, the exterior bottle may have in each vent a plug-shaped valve. Alternatively, the interior bag portions facing the vents may serve themselves as check valves for temporarily closing same from inside. A negative pressure produced in between the exterior bottle and interior bag will push such bag portions inwardly to open those vents. It is preferable that those portions of interior bag return to normal position to tightly close said vents again. Alternatively, the vents may be formed in the bottle mouth and reversibly closed with corresponding areas of a thickened mouth portion (viz., outlet opening) of interior bag. Shrinkage of the bag's barrel region in response to progressive decrease in volume of the liquid content will not be hindered in this case, too.

In still another fashion employable herein, such check valves are dispensed with by rendering the diameter of each vent in the exterior bottle much smaller than that of a discharging channel formed through the interior bag. In this case, the exterior bottle can be squeezed to compress the air intervening between it and the interior bag, thereby depressing the latter to batch-wise exude (and drop) the liquid content through its discharging channel. In detail, each vent is made as a small orifice having a diameter for instance of about 0.1 mm to 0.5 mm. A compression rate of squeezing the exterior bottle in this case is rendered higher than an exhaustion rate at which the intervening air will be forced out through the vents. Therefore, said intervening air is compressed to centripetally depress the interior bag to shrink and thereby discharge the liquid content through the channel formed in the plug assembly. Effective cross-sectional area of this discharging channel is made so larger than those of vents that the flow resistance against the liquid content being exuded is much lower than that which will be imparted to the air being exhausted.

The invention can be practiced to provide not only the eyedropper or the like pharmaceutical dispenser, but also and alternatively a cosmetics container, a detergent container, or any other appropriate one. As already discussed above, the container may substantially consist of a delaminating bottle composed of an exterior bottle and an interior bag. A flexible nozzle is fixed in the mouth of such a delaminating bottle, and further a cap may detachably be attached to the mouth so as to cover the nozzle and closely contact the opening end of an outlet hole formed in and through said nozzle.

The flexible nozzle may either be constructed as an integral one-piece or be composed of two or more parts combined with each other. The nozzle may be fitted in or on the bottle mouth, wherein a small-diameter cylindrical portion is formed as one principal part of this nozzle. Such a nozzle portion protrudes upwards, with the bore thereof serving as a discharging channel. Desirably, the flexible nozzle may have a cylindrical base fitted in the bottle mouth, in addition to the upwardly protruding small-diameter portion and an intermediate portion continuing from those two former portions and functioning as an elastic deformation region. The outlet hole is subject to inward and outward displacement as a result of axial movement of the intermediate portion. This intermediate portion can be bellows-shaped, dome-shaped or be of any other proper shape.

Now, the best modes of carrying out the present invention will be described referring to the drawings.

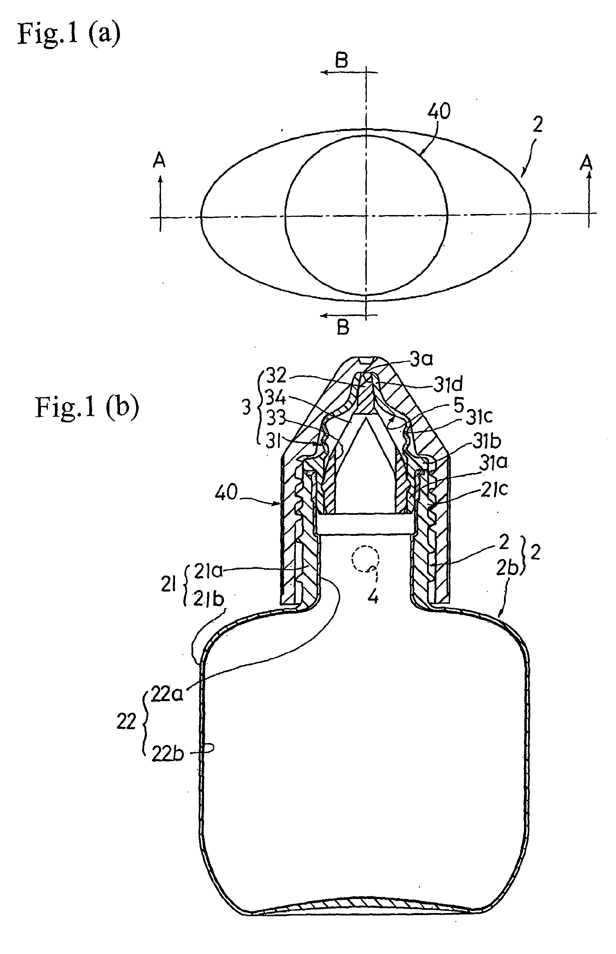

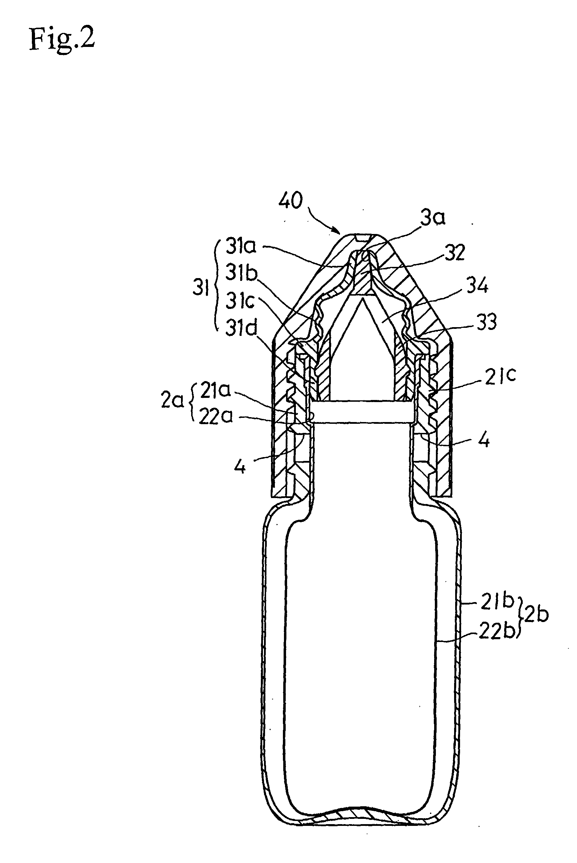

Figs. 1(a), 1(b) and 2 show an eyedropper 1 as the dispensing container provided in a first embodiment. One of principal parts of this eyedropper 1 is a laminated bottle 2 capable of exfoliation and comprising a mouth 2a and a barrel 2b. A plug assembly 3 having an outlet hole 3a formed therethrough is secured to the mouth 2a, together with a cap 40. The outlet hole 3a normally remains closed, but can be opened if a user removes the cap 40 and then puts the container 1 upside down to subsequently squeeze the barrel 2b. When the user operates the container in this way, eye drops (viz., drops of a liquid content) retained in the bottle 2 will be dispensed (in a dripping manner) through the outlet hole 3a of plug assembly 3. Once he or she stops depressing the barrel 2b, this hole 3a will be closed again.

The delaminating bottle 2 consists of an exterior bottle 21 as the outer layer and an interior bag 22 as the inner layer laminated on the former layer. Since the main part of dispensing container in this embodiment is such a laminated bottle, the exterior bottle (or bottle body) will hereinafter be simply referred to as an 'outer layer', with the interior bag being referred to as an 'inner layer'. However, it will be understood that any other containers whose bottle body and interior bag are not laminated one on another but merely adjoined to each other are also included in the scope of present invention.

Both the outer and inner layers 21 and 22 have their cylindrical mouth portions 21a and 22a in addition to their barrel portions 21b and 22b elliptic in transverse cross section. In other words, the mouth 2a consists of these mouth portions 21a and 22a, with the barrel 2b consisting of these barrel portions 21b and 22b. The outer layer 21 is made of a relatively harder plastics such as a PET or an EVOH, whilst the inner layer 22 is made of a typically softer plastics (for example a polyolefin such as PE) ready to be delaminated from the outer layer. The outer layer barrel portion 21b is capable of elastic squeezing when depressed in the direction of its minor axis, with the inner layer mouth portion 22a defining an outlet opening in the interior bag.

The upper end of the inner layer mouth portion 22a is firmly secured to that of the outer layer mouth portion 21a. Inner periphery of the latter portion 21a may be knurled in axial direction to have longitudinal grooves and ridges at angular intervals. In this case, the inner layer mouth portion 22a is protected from any inadvertent rotation relative to the outer layer mouth portion 21a.

For introduction of ambient air into a clearance or gap between the outer and inner layer barrel portions 21b and 22b, at least one vent(s) 4 is(are) formed in the outer layer mouth portion 21a. Two such vents in the present embodiment face one another in diametrical direction. Each vent 4 penetrates only the outer layer 21 transversely, not to injure the inner layer 22. Formed on and around the outer periphery of outer layer mouth portion 21a is a screw thread 21c.

The inner layer barrel portion 22b is a film-like part and is therefore ready to shrink as the volume of liquid content decreases during use of this container. The mouth portion 22a of inner layer made noticeably thicker than its barrel portion 22b can restore its really cylindrical configuration at every cycle of operation of this container. Although the inner layer barrel portion 22b may not be seen clearly in the drawings due to its extreme small thickness, the inner periphery of outer barrel portion 21b is laminated on and fully covered with such a thin inner barrel portion.

The inner mouth portion 22a is normally at its position to close from inside the vents 4 in outer mouth portion 21a. Thus, the inner portion 22a works as closing members for the respective vents 4 in one of its states. However, said inner portion will serve on the other hand as a check valve, because atmospheric pressure urges it to open those intakes when a negative pressure arises in between the barrel portions 21b and 22b.

Though not shown in the drawings, the inner layer's 22 bottom may have at its center a latching member formed integral therewith. This member is firmly engaged with the bottom center of outer layer 21 to thereby protect the inner layer from curling upwards.

The plug assembly 3 comprises a generally cylindrical flexible nozzle 31 having the outlet hole 3a to be closed with a rod-shaped valve body (as a closer) 32. A cylindrical sleeve 33 possessed by the plug assembly 3 functions to secure both the nozzle 31 and valve body 32 to the bottle mouth. Connection ribs 34 are formed integral with both the sleeve 33 and valve body 32. These three parts 32, 33 and 34 constitute a one-piece integral body made of a suitable plastics. The short rod-shaped closer 32 extending coaxially with the bottle mouth 2a is disposed out of the opened end of this mouth.

The flexible nozzle 31 has a cylindrical base 31a fitted in the bottle mouth 2a, and a flange 31b in contact with the upper end of mouth 2a. The nozzle further has a bellows-shaped intermediate portion 31c and a nozzle portion 31d continuing therefrom longitudinally and outwardly of the bottle. The bellows-shaped portion 31c ready to elastic deformation does continue from the base 31a longitudinally in outward ('upward' in the drawings) direction of the bottle. Such a nozzle 31 is a one-piece made of a flexible material such as a silicone rubber. The flexible nozzle 31 has an axis in parallel with the axis of bottle mouth 2a, and in the illustrated example, the nozzle portion 31d lies coaxial with said mouth 2a. The outlet hole 3a mentioned above is a bore of nozzle portion 31d, and receives the rod-shaped valve body (as the closer) 32 inserted in this hole to serve as a closing member. So long as the outlet hole 3a remains closed, its open end stands in flush with the upper end of valve body 32. Due to this feature, an overall outer and upper configuration of the flexible nozzle 31 and valve body 32 closing it and retained therein does coincide with and closely contact the inner and upper periphery of the cap 40. The sleeve 33 disposed inside and all around the flexible nozzle's base 31a presses it outwards in a centrifugal direction to the inner periphery of bottle mouth 2a, whereby air-tightness and liquid-tightness are ensured between the base 31a and bottle mouth 2a firmly adjoined thereto.

The elastically deforming intermediate zone 31c of flexible nozzle 31 is made considerably thinner than all of the remaining portions thereof, that is the base 31a, flange 31b and nozzle portion 31d. Such a thinned and bellows-shaped zone 31c is prone to make an elastic elongation in axial direction. This motion will bring about outward displacement of the outlet hole 3a a distance enough to disengage from the rod-shaped valve body or closer 32. With the intermediate zone 31c simultaneously moving in axial direction towards its inner home position, the outlet hole 3a will restore its inner normal position so as to be closed again with the closer 32.

Such an elastic deformation and displacement of the portions of flexible nozzle 31 occurs as the internal pressure of the inner layer 22 rises by operation of this container. In detail, the inner layer's pressure raised above atmospheric pressure will act on a pressure-receiving area 5. This area facing downwards and formed as an upper and inner peripheral area of the elastically deforming intermediate portion 31c will consequently force the nozzle portion 31d outwards away from the barrel. A negative pressure produced in the inner layer 22 during introduction of ambient air through the intakes 4 will pull back the pressure-receiving area 5, returning the nozzle portion 31d to its inner (lower) position, thus letting the flexible nozzle 31 as a whole recover its normal configuration. As will now be apparent, the pressure-receiving area 5 is always in a fluid communication with the interior of inner layer 22 and thus exposed to the internal pressure thereof, whether the outlet hole 3a is opened or closed. Elastic forward deformation of flexible nozzle 31 will thus move the outlet hole 3a outwardly along the rod-shaped closer 32 towards or beyond a distal end thereof, when this hole is opened. Reverse deformation of said nozzle will likewise displace the outlet hole 3a but towards a proximal end of said closer, when this hole is closed.

The cap 40 is releasably screwed on the outer periphery of the outer layer mouth portion 21a. During non-use of this container 1, the cap will continue to seal the outer periphery of flexible nozzle 31 in order to protect it from dusts and bacteria. Inner shape of the cap 40 is analogous with the outer contour of nozzle 31 in its non-deformed state. The inner top surface of this cap lies in a plane including both the distal ends of nozzle portion 31d and rod-shaped closer 32. This means that, after use of the container, the cap 40 will and can be put on it to drive backwards the nozzle portion 31d along the closer until the outlet hole 3a is perfectly closed with the closer 32. The cap 40 tightly mounted in this manner will force a backward motion of nozzle portion 31d, effectively repelling any residual amount of liquid content around the forward end of outlet hole 3a. Any noticeable amount of residue of eye drops will not be left around and outside the outlet hole, thus protecting a fresh dose of the liquid content from contamination when using later the container again.

When the user wants to exude and dispense eye drops through the outlet hole 3a of eyedropper 1 of the present embodiment, he or she has to remove at first the cap 40 and then cause the bottle 2 to take a position reversed upside-down. Subsequently, he or she depresses the barrel 2b in a centripetal direction along its minor axis. Internal pressure of the inner layer 22 will be raised in this way to elastically deform the flexible nozzle 31 and open the outlet hole, supplying 'eye drops' from the distal tip of nozzle portion 31d. If and when the user simply ceases to depress the delaminating bottle 2, the outer layer will almost instantly restore its normal shape. However, any amount of ambient air will not flow into the inner layer 22 through the outlet hole 3a, because this hole 3a is then in a closed state due to elastic recovery of natural shape of the flexible nozzle 31. The inner layer does not restore its normal un-shrunk shape, even if the liquid content is consumed further and later when demanded. In contrast, the outer layer 21 will be allowed to take its normal shape again, giving rise to a certain degree of negative pressure between the outer and inner layer barrel portions 21b and 22b. Consequently, atmospheric pressure deforms in a centripetal direction the inner layer mouth portion's 22a area facing the vents 4, thereby opening them in outer layer 21 so as to permit inflow of a certain amount of ambient air through said intakes. It is to be noted here that the negative pressure still remains within the interior bag 22 for a time. Such a remaining negative pressure will act on the pressure-receiving area 5, to thereby cause the flexible nozzle to restore its natural shape until it perfectly closes the outlet hole 3a. As a sufficient amount of ambient air sucked inwards through the vents 4 permits recovery of natural shape of the outer layer barrel portion 21b, the inner layer mouth portion 22a will spontaneously regain its home position. Any extent of temporary deformation will not be left in this mouth portion 22a, but it surely closes the vents 4.

Later, the user may operate again this container by depressing its bottle 2, whose inner layer mouth portion 22a has been and is still closing the vents 4. The air retained in the gap between the outer and inner layer barrel portions 21b and 22b can not escape out by such as a resumed operation, but will be compressed by depressing the outer barrel portion 21b. The air thus compressed again within the outer barrel will work to squeeze the inner barrel portion 22b and dispense the eye drops in the exuding manner as detailed above.

It will now be apparent that the dispensing container of the described embodiment has a flexible nozzle 31 and a rod-shaped valve body (viz., closer) cooperating therewith as if they were component parts of a check valve. Ambient air is excluded from the interior of inner layer 22, and therefore it will shrink to protect the liquid content from contamination with bacteria that are possibly present in the ambient air. On the other hand, the vents 4 enable ambient air to flow in between the outer and inner layers 21 and 22. Depression of the outer layer 21 will not produce any permanent deformation thereof after this layer is released from the user's fingers or the like means having been squeezing it. Elastic recovery of the outer layer 21 is ensured so that its natural shape can last until the liquid content is thoroughly consumed to empty the inner layer 22. Thus, this dispensing container 1 will continue to stand unchanged in appearance from outside after every cycle of operation from start to end of use, highly convenient to users. The liquid content of inner layer 22 is shut from ambient air so that any preservatives for sterilization need no longer be added to the content. Any appropriate materials may be used to form the inner and outer layers, taking into account requirements which they have to meet in respect of transparency, gas barrier property and water permeation. This dispensing container affords optimal features widely in many uses, for example as an eyedropper, a detergent container or the like.

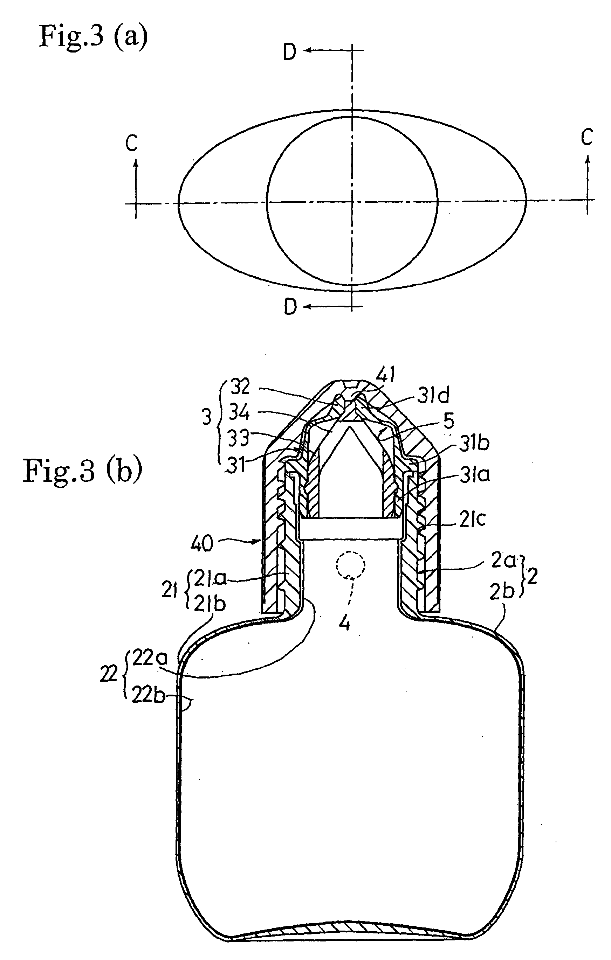

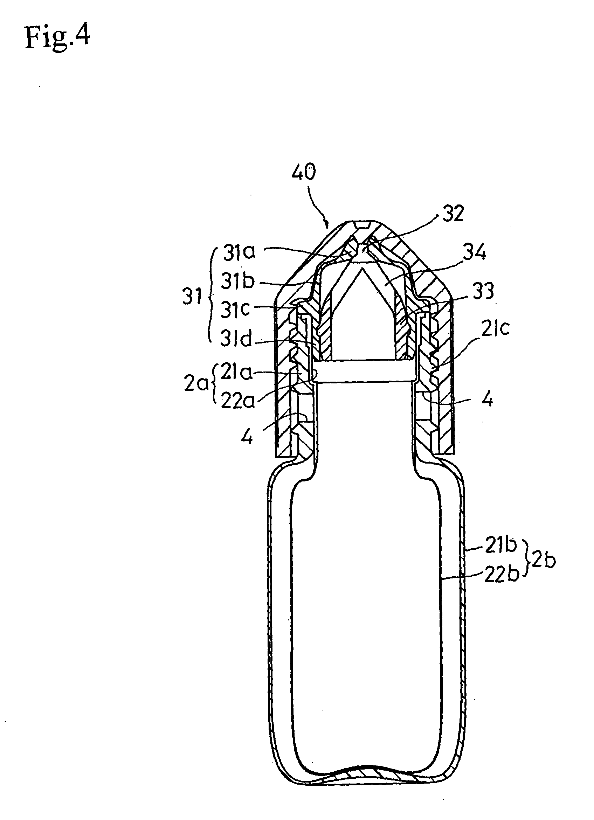

Figs. 3(a) to 4 show a dispensing container 1 provided in a second embodiment. Its structural elements that are the same as or similar to those described above in the first embodiment will not be discussed again, bur merely be denoted with the same reference numerals. Only elements and functions peculiar to the second embodiment will be described below.

The flexible nozzle 31 comprises, for the purpose of its elastic deformation, an intermediate portion 31c that is dome-shaped. This elastic intermediate portion can be stretched forwards in order to bring the outlet hole 3a into its position disengaged from the rod-shaped closer 32. A recess will appear ahead the distal end face of the closer 32 and in the forward end region of outlet hole 3a. A little residual amount of liquid content will stay in this recess, unless the cap 40 has a portion fitting in it. Therefore, a lug 41 protruding downwards is formed in this embodiment to be integral with the cap at its center of ceiling. With such a cap 40 being put on the bottle mouth, the lug 41 will completely repel the residue out of said recess so as to avoid the problem of contamination.

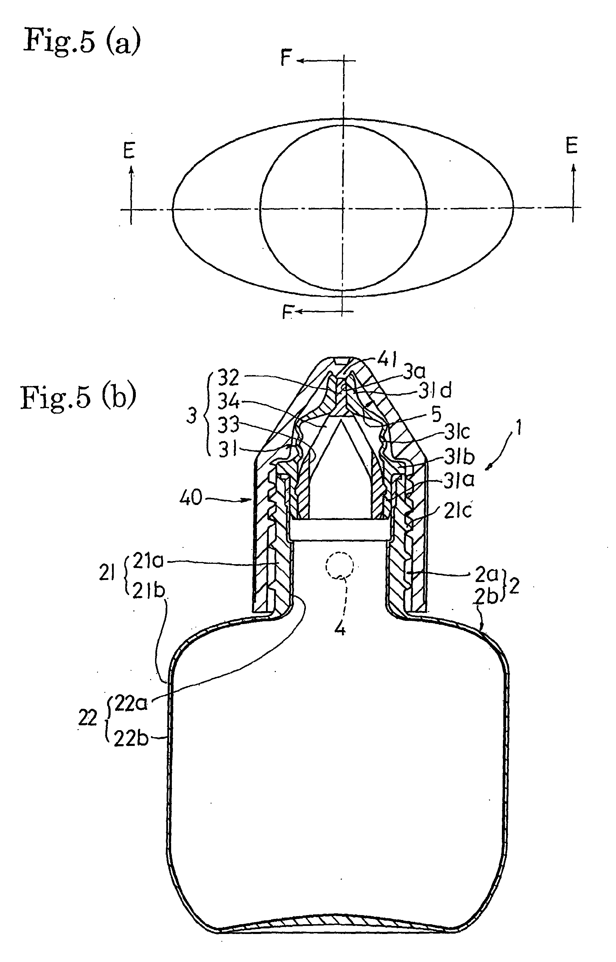

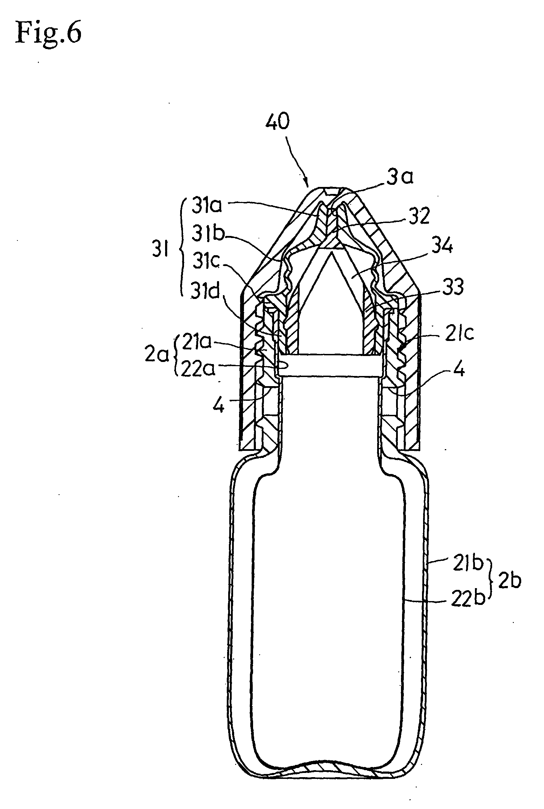

Figs. 5(a) to 6 show still another dispensing container 1 in a third embodiment of the present invention. Its nozzle portion 31d rendered thicker than that in the first embodiment has an improved dimensional stability. The outlet hole 3a has a noticeably reduced diameter, and correspondingly, the rod-shaped closer 32 in this container is much thinner and more elongated. Other structural elements not differing from those in the first and second embodiments are denoted with the same reference numerals, and description thereof is not repeated.

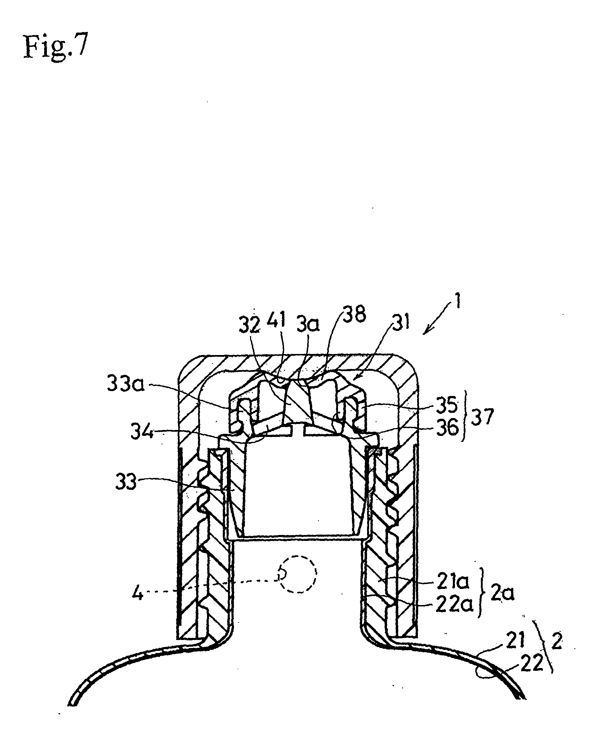

Fig. 7 illustrates a dispensing container 1 provided in a fourth embodiment. Also, its structural elements that are the same as or similar to those in the first embodiment are merely denoted with the same reference numerals. Only details of other elements peculiar to the fourth embodiment will be given here.

The flexible nozzle 31 in this container 1 is composed of two portions, that is a cylindrical base 37 and an elastically deforming portion 38 made integral therewith and arc-shaped in vertical cross section. The base or base 37 is formed as a kind of double cylinder whose outer and large-diameter section 35 extends coaxial with and surrounds an inner and small-diameter section 36. The deforming portion 38 continuing from the top end of said base 37 may be regarded as a complex of the nozzle portion 31d and intermediate portion 31c integral therewith in the preceding embodiments. Such a flexible nozzle 31 fixed on the upwardly protruding top end 33a of a sleeve 33 is not directly attached to the bottle mouth 2a but indirectly connected thereto by the sleeve 33 in this embodiment. An outlet hole 3a is a central round opening of the elastically deforming portion 38. This portion 38 normally takes a convex position bulging inwardly of the bottle as shown in Fig. 7, so that its outlet hole 3a remains closed with the closer 32. When pressure in the bottle rises, the deforming portion's inner face (viz., lower face in the drawings and serving as if it were a pressure-receiving area 5 in the former embodiments) will be pressed up to deform itself elastically to take a reversed convex shape facing outwards. In this state, the outlet hole 3a is raised and displaced away from the closer 32 so as to open. On the other hand, the rod-shaped closer 32 in this embodiment gradually increases its diameter towards its basal lower' end. When any degree of negative pressure is produced inside the bottle, the central region of elastically deforming portion 38 then at its position shown in Fig. 7 will slide down ('inwards') along the closer. However, the outlet hole 3a will remain sealed with the closer 32 not to undesirably and inadvertently open to any extent.

The large- and small-diameter sections 35 and 36 of the nozzle base 37 firmly grip and sandwich the upwardly protruding end 33a of the sleeve 33, from outside and inside. Thus, an airtight and liquid-tight connection is provided between these members connected to each other.

The present invention is not delimited to the foregoing embodiments or examples but may be modified within the spirit and scope set forth in the appendant claims.

In summary, the present container is of a relatively simple structure, but can nevertheless dispense a liquid pharmaceutical content or the like, without permitting any inflow of ambient air into the interior bag. None of ordinary bacteria often present in the environment will growth within this bag, even if preservatives are not added at all to the liquid content. Once internal pressure of the bag returns to atmospheric pressure, the flexible nozzle temporarily deformed to have its outlet hole displaced up away from the container body will regain its normal position. In other words, the outlet hole moves inwardly towards the container body until closed again with the closer. With the outlet hole being closed in this manner, the amount of liquid content that has been dispensed through the outlet hole would have dropped off the nozzle almost in its entirety. Thus, any notice-able residual amount of the liquid once dispensed will not stay outside and around said hole, causing no problem of contamination of such a residue.

## Claims

1. A dispensing container comprising a bottle, an interior bag of the bottle and a plug assembly,
the bottle comprising a barrel and a mouth,
the bag being containable a liquid content,
the bag being placed in the barrel of the bottle,
the bag having an outlet opening for dispensing the liquid content,
the plug assembly being disposed in the mouth of said bottle,
the outlet opening being connected to the mouth of said bottle,
the bottle having at least one vent for intake of ambient air in between the bottle and the bag,
the plug assembly comprising a nozzle having an outlet hole for dispensing the liquid content out of the bottle, and a closer capable of closing the outlet hole,
the closer being fixed in the mouth of the bottle, and
the nozzle being capable of elastic deformation away from a normal position such that the outlet hole is displaced outwards relative to the closer in response to a pressure raised in the bag and disengage from the closer.

2. A dispensing container as defined in claim 1, wherein the closer is a rod-shaped member inserted in and closing the outlet hole, and the nozzle has an inner surface always in communication with an interior of the interior bag such that a negative pressure arising therein will force the flexible nozzle backwards toward the normal position.

3. A dispensing container as defined in claim 1, wherein the plug assembly further comprises a sleeve made integral with the closer, the nozzle is formed of a pliable and elastic material such as a rubber, the nozzle has a cylindrical base fitted in the mouth of the bottle, and the sleeve is placed inside the base so as to press the base all around to an inner periphery of the mouth to thereby ensure an airtight and liquid-tight connection thereof with the nozzle.

4. A dispensing container as defined in claim 1, wherein the plug assembly further comprises a sleeve made integral with the closer and fitted in the mouth of the bottle, the nozzle formed of a pliable and elastic material such as a rubber has a cylindrical base, and the base surrounds the closer and is in an airtight and liquid-tight contact with the sleeve.

5. A dispensing container as defined in claim 1, further comprising a cap detachably mounted on the mouth of the bottle and having an inner periphery adjacent to top of the cap, wherein the periphery is substantially in conformity with upper end regions which the nozzle and the closer define in state what the cap is mounted on the mouth and the cap in the mounted state does closely contact end faces of the upper end regions.

6. A dispensing container as defined in claim 1, wherein the barrel of the bottle is capable of elastic deformation to reduce its internal volume and to thereby compress the interior bag and elastically deform the nozzle, whereby the nozzle outlet hole thus opened will permit the liquid content to exude out therethrough, and when the barrel returns to its un-deformed normal position causing the nozzle towards its normal closed position, the vents allow ambient air to flow in between the exterior bottle and the interior bag.

7. A dispensing container as defined in claim 1, wherein an open end of the outlet opening is closely connected all around to an open end of the mouth of the bottle.

8. A dispensing container as defined in claim 1, wherein the outlet opening is adhered all around to an inner periphery of the mouth to thereby inhibit deformation of the adhered portion of the interior bag.

9. A dispensing container as defined in claim 1, wherein the at least one vent is disposed in the mouth of the bottle.

10. A dispensing container as defined in claim 1, further comprising a check valve that allows inflow of the ambient air into a clearance between the exterior bottle and the interior bag, but prevents outflow of the ambient air having entered the clearance.

11. A dispensing container as defined in claim 1, wherein the closer is fixed in place relative to the mouth of the bottle.

12. A dispensing container as defined in claim 1, wherein the nozzle comprises a cylindrical base fitted in the mouth, a nozzle portion having a bore as the outlet hole, and an elastically deformable intermediate portion provided between the nozzle portion and the base in a connected row arrangement such that elastic deformation of the intermediate portion displaces the outlet hole outwardly of the container.

13. A dispensing container as defined in claim 12, wherein the elastically deformable intermediate portion is bellows-shaped.

14. A dispensing container as defined in claim 12, wherein the elastically deforming intermediate portion is dome-shaped.

15. A dispensing container as defined in claim 1, wherein the nozzle comprises a cylindrical base and an arc-shaped elastically deformable portion connected to an upper end of the base, the base being a double cylinder comprising a first cylindrical section and a second cylindrical section smaller than the first cylindrical section in diameter.
